(19)

Europäisches Patentamt

European Patent Office

Office européen des brevets

(11) **EP 1 930 020 B1**

(12) **EUROPEAN PATENT SPECIFICATION**

(45) Date of publication and mention of the grant of the patent:
**01.06.2011 Bulletin 2011/22**

(21) Application number: **06775453.1**

(22) Date of filing: **22.08.2006**

(51) Int Cl.:
*A61K 36/804* (2006.01)    *A61K 36/74* (2006.01)
*A61K 36/71* (2006.01)    *A61K 36/66* (2006.01)
*A61K 36/536* (2006.01)    *A61K 36/486* (2006.01)
*A61K 36/482* (2006.01)    *A61K 36/236* (2006.01)
*A61K 36/232* (2006.01)    *A61K 35/56* (2006.01)
*A61P 25/04* (2006.01)

(86) International application number:
**PCT/CN2006/002135**

(87) International publication number:
**WO 2007/022710 (01.03.2007 Gazette 2007/09)**

(54) **CHINESE MEDICINAL COMPOSITIONS FOR TREATING HEADACHE, FORMULATIONS AND PROCESSES FOR PREPARATION THEREOF**

CHINESISCHE MEDIZINISCHE ZUSAMMENSETZUNGEN ZUR BEHANLDUNG VON KOPFSCHMERZEN, FORMULIERUNGEN UND VERFAHREN ZU IHRER HERSTELLUNG

COMPOSITIONS A BASE DE MEDECINE CHINOISE POUR LE TRAITEMENT DE CEPHALEES, FORMULATIONS ET PROCEDES DE PREPARATION

(84) Designated Contracting States:
**DE FR GB**

(30) Priority: **24.08.2005   CN 200510014828**
**24.08.2005   CN 200510014829**

(43) Date of publication of application:
**11.06.2008   Bulletin 2008/24**

(73) Proprietor: **Tianjin Tasly Pharmaceutical Co., Ltd. Beichen District**
**Tianjin 300-402 (CN)**

(72) Inventors:
 • **WU, Naifeng**
  **Tianjin 300402 (CN)**
 • **YAN, Xijun**
  **Tianjin 300402 (CN)**
 • **ZHANG, Shunnan**
  **Tianjin 300402 (CN)**
 • **YANG, Jianhui**
  **Tianjin 300402 (CN)**
 • **ZHANG, Hongpo**
  **Tianjin 300402 (CN)**
 • **DONG, Lina**
  **Tianjin 300402 (CN)**
 • **LIU, Tong**
  **Tianjin 300402 (CN)**

 • **SUN, Yan**
  **Tianjin 300402 (CN)**
 • **ZHOU, Lihong**
  **Tianjin 300402 (CN)**
 • **WANG, Hui**
  **Tianjin 300402 (CN)**
 • **MA, Jie**
  **Tianjin 300402 (CN)**
 • **YE, Zhengliang**
  **Tianjin 300402 (CN)**

(74) Representative: **Gillet, Raphaëlle et al**
**Cabinet Beau de Loménie**
**158, rue de l'Université**
**75340 Paris Cedex 07 (FR)**

(56) References cited:
**CN-A- 1 073 874    CN-A- 1 160 556**
**CN-A- 1 631 399    CN-C- 1 047 938**

 • **SCHANEBERG B T ET AL: "Analysis of products suspected of containing Aristolochia or Asarum species" JOURNAL OF ETHNOPHARMACOLOGY, ELSEVIER SCIENTIFIC PUBLISHERS LTD, IE, vol. 94, no. 2-3, 1 October 2004 (2004-10-01), pages 245-249, XP004533046 ISSN: 0378-8741**

- Anonymous: "List of products category M" Huaihua Longyuan Medicine CO., LTD, [Online] 31 December 2009 (2009-12-31), pages 1-10, XP002565709 Retrieved from the Internet: URL: http://www.lonyuan.net/herb-supply/her b-price/product-price/13-3-m.htm> [retrieved on 2010-12-31]

- ZHOU Z. ET AL. INFO OF TCM vol. 20, no. 6, 2003, pages 30 - 31, XP008077169
- CHEN H. ET AL. CANTON PHARM. vol. 10, no. 6, 2000, pages 28 - 30

**Description**

**Field of the invention**

The present invention relates to the field of Chinese medicine. More specifically, the invention relates to Chinese medicinal compositions for treating headache, formulations and processes for preparation thereof.

**Background of the invention**

Chinese Patent No. ZL 93100050.5 disclosed a pharmaceutical composition comprising *Radix Angelicae Sinensis* (Dang gui) and *Rhizoma Chuanxiong* (Chuan xiong), etc., which is the fruits of long-term clinical practice under the guidance of Chinese medicine theory, having the therapeutic effects of treating headache caused by inner damage. Clinically, it can be applied to treat several headache diseases, such as the angioneurotic headache, migraine, and some symptom like dizziness and headache etc. caused by hypertension. According to the proportion of this recipe, the composition can be prepared into any type of pharmaceutically acceptable dosage forms, wherein the granule is produced by Tianjin Tasly Pharmaceutical Co. Ltd., named as "Yang Xue Qing Nao Granule". The functions and indications approved by the authorities are nourishing blood and calming liver, activating blood and removing obstruction in channels, used for diverse types of headache elicited by blood deficiency and hyperactivity of liver, traumatic cranial nerve syndrome, dizziness, irritation, and insomnia and dreamful sleep. In clinic, it has been usually used for treatment headache caused by blood deficiency, blood stasis, and deficiency of Yin and hyperactivity of Yang. Since coming into the market, Yang Xue Qing Nao Granule has enjoyed wide popularity among the patients due to its reassured therapeutic effects.

While the Yang Xue Qing Nao Granule has got generally-recognized clinically, the usage of *Herba Asari* (Xi xin) herein is in places needing further discussion.

Long since, the dosage of *Herba Asari* in recipe is a problem widely disputed. As an old saying goes that the dosage of *Herba Asari* is less than *Qian,* an old weight unit in China, it therefore suffices to prove that the dosage of *Herba* Asari should pay more attentions.

*Herba Asari* is one of very common-used Chinese medicines, which is firstly recorded in the ShenNong BenCao jing (Shen Nong's Classic of Chinese Medicine). It is pungent and warm in nature, having a little toxicity, manifesting its therapeutic effects in lung, kidney and heart and acting as a function to relieve exterior syndrome and dispel wind, relieve pain and clear passage, and warming the lung and reducing phlegm. Clinically, it is suitable for the treatment of many diseases, such as the wind-cold type of cold, headache, toothache, rheumatoid arthralgia disease (Bi diseases), etc.

Modern pharmacology study showed that the toxicity of *Herba Asari* lied mainly with its volatile oils fractions. A large amount of volatile oil may make animals excited, further paralyzing voluntary motion, gradually weakening respiratory movement, eliminating the physiological reflections and ending up with death because of the paralysation of respiratory [Ding Tao, The adverse reaction of Chinese medicine and its prevention [M], China Press of Chinese Medicine, Beijing 1992, p85].

The granule is a new dosage form which is developed from Chongji (an instant dosage form which is dissolved with water before administration). The recent decade witnesses its rapid progress. Not only does the granule keep the Chongji's features of convenient administration, preservation and transportation, but also overcomes Chongji's defects of high-content of sugar, making it suitable for the old and diabetics. On the other hand, due to the decrease in the amount of the adjuvants in the granule, the size thereof is much smaller, and the active ingredient therein is higher than that of the Chongj. However, the granule contains more extractum and much less additives, thus makes the granule easily absorb dampness, conglomerate and change its color etc. Meanwhile, the granule is extreme bitterness in taste and some may either produce strong stimulating effects on stomach or tend to be damaged in stomach. All these problems of the granule need to be solved.

After long-term practice, the inventors discovered a new kind of concentrated pills; said concentrated pills are prepared by a process as follows: extracting the Chinese medicines properly, adding appropriate adjuvants to prepare into pills, and the pills are prepared into coated concentrated pills by film-coating technique. The concentrated pills can avoid many problems, such as taking large dosage for one time, absorbing dampness, bitterness when taken with water and unpleasant taste such as sour and astringent.

**Detailed description of this invention**

The objective of the invention is to optimize the recipe in the prior arts and provide a *Herba Asari*-free composition for treating headache.

The composition of this invention for treating headache is prepared by the following medicinal materials in proportion by weight of: 5-15 wt% of *Radix Angelicae Sinensis* (Dang gui), 5-15 wt% of *Rhizoma Chuanxiong* (Chuan xiong), 2-10 wt% of *Radix Paeoniae alba* (Bai shao), 2-10 wt% of *Radix Rehmanniae Preparata* (Shu dihuang), 7-17 wt% of *Ramulus Uncariae cum Uncis* (Gou teng), 7-17 wt% of *Caulis Spatholobi* (Ji Xueteng), 7-17 wt% of *Spica Prunellae* (Xia Kucao), 7-17 wt% of *Semen Cassiae* (Jue mingzi), 7-17 wt% of *Concha Margaritifera Usta* (Zhen zhumu) and 3-10 wt% of *Rhizoma Corydalis* (Yuan hu).

Preferably, the composition of this invention is prepared by the following medicinal materials in proportion by weight of: 5-8 wt% of *Radix Angelicae Sinensis,* 5-8 wt% of *Rhizoma Chuanxiong,* 3-7 wt% of *Radix Paeoniae alba,* 3-7 wt% of *Radix Rehmanniae Preparata,* 10-15 wt% of *Ramulus Uncariae cum Uncis,* 10-15 wt% of *Caulis Spatholobi,* 10-15 wt% of *Spica Prunellae,* 10-15 wt% of *Semen Cassiae,* 10-15 wt% of *Concha Margaritifera Usta* and 3-10 wt% of *Rhizoma Corydalis.*

Most preferably, the composition of this invention is prepared by the following medicinal materials in proportion by weight of: 6.85 wt% of *Radix Angelicae Sinensis,* 6.85 wt% of *Rhizoma Chuanxiong,* 5.5 wt% of *Radix Paeoniae alba,* 5.5 wt% of *Radix Rehmanniae Preparata,* 13.69 wt% of *Ramulus Uncariae cum Uncis,* 13.69 wt% of *Caulis Spatholobi,* 13.69 wt% of *Spica Prunellae,* 13.69 wt% of *Semen Cassiae,* 13.69 wt% of *Concha Margaritifera Usta* and 6.85 wt% of *Rhizoma Corydalis.*

According to the composition of this invention, it can be prepared into decoctions by the method of Chinese medicine and taken directly, or can be prepared into any one of pharmaceutically acceptable formulations by steps of extracting the medicinal materials to give the extract, and mixing it with appropriate adjuvants.

Thus, another objective of this invention is to provide conventional pharmaceutical formulations comprising the composition, and the pharmaceutical formulations are produced by a conventional pharmaceutical method in the pharmaceutical field and prepared from the medicinal materials in the proportion mentioned-above and common pharmaceutically acceptable carries.

The pharmaceutical formulations comprising the composition of this invention may be a solid and liquid formulations suitable to be administered orally as well as a sustained and controlled-release formulations, etc. The solid formulations suitable to be administered orally may be tablets, capsules, granules, pills, powder and various sustained and controlled-release formulations, etc. The preferable formulations are concentrated pills, more preferable formulations film-coating pills, and the most preferable are stomach-soluble film-coating pills.

The stomach-soluble film-coating pill comprising the composition of this invention does not have the defects of the granules, such as high-content of extractum, some problems during the preservation and transportation like easily absorbing dampness, conglomerate, and change in color, and extreme bitterness in taste, strong stimulating effects and their active ingredient tend to be damaged in stomach. The results of experiments show that the dissolution curve of concentrated pills of the invention, especially the stomach-soluble film-coating pills (hereinafter also refers to "Yang Xue Qing Nao Pill") is similar to that of the known granules in the prior art (hereinafter also refers to "Yang Xue Qing Nao Granule"), indicating that the dissolution *in vitro* of the two drugs is identical.

The pills of the invention may contain common pharmaceutical adjuvants and binding agents. The adjuvants may be selected from the group consisting of: dextrin, starch, low-substituted hydroxypropyl cellulose and microcrystaline cellulose, and microcrystaline cellulose is preferable. The amount of adjuvants accounts for more than 25% of the weight of dried medicinal materials powder, preferably, 28-35%. The binding agents may be selected from the group consisting of: water, 20-30% (v/v) ethanol aqueous solution, and honey aqueous solution, preferably, the binding agent is water. The amount of the binding agent is appropriate when the optional binding effect achieved.

The term "blank pills" of the invention means the conventional pills in pharmaceutical field, and especially refers to the non-coating pills.

The term "pills" or "concentrated pills" of the invention means the pills generally prepared by mixing the concentrated extract of the medicinal materials with adjuvants.

The term "relative density" of the invention means the density value with 1g/cm$^3$ of water in 4°C as the reference density.

Another objective of this invention is to provide a method for preparation of the composition.

According to the invention, the composition may be either prepared into decoctions by the method of Chinese medicine and taken directly, or can be prepared into any one of pharmaceutically acceptable formulations by steps of extracting the medicinal materials to give the extract, and mixing it with appropriate adjuvants.

According to the composition of this invention, *Radix Angelicae Sinensis, Rhizoma Chuanxiong* and *Radix Paeoniae alba* may be extracted alone or in any combinations to give a medicinal materials extract by the process, but not limited to, of extracting with water and then precipitated with ethanol; ultrasound extraction, or supercritical extraction, etc.

For comprehending, the embodiments of the extractive methods of the four medicinal materials, namely, *Radix Angelicae Sinensis, Rhizoma Chuanxiong, Rhizoma Corydalis* and *Radix Paeoniae alba* are described as follows, but not for limitation of the invention.

The method of extracting *Radix Paeoniae alba* by ultrasound extraction: *Radix Paeoniae alba* is taken and pulverized into powders, into which water is added and extracted by ultrasound extraction to give the *Radix Paeoniae alba* extract.

The method of extracting *Radix Paeoniae alba, Rhizoma Chuanxiong, Rhizoma Corydalis* and *Radix Angelicae Sinensis* with ethanol: the medicinal materials are taken and pulverized into powders, into which ethanol is added and refluxed. The obtained extraction is concentrated to recover ethanol, and further concentrated to give the extract.

The method of extracting *Radix Angelicae Sinensis* by supercritical extraction: *Radix Angelicae Sinensis* is taken and extracted by $CO_2$ supercritical extraction under appropriate conditions. After being filtered, the *Radix Angelicae Sinensis* extract is given.

The method of extracting *Radix Angelicae Sinensis* with water and resin: the precipitate, which is produced by extracting *Radix Angelicae Sinensis* with water and then precipitated with ethanol, is dissolved with distilled water by heating; its pH value is adjusted with saturated calcium hydroxide solution, and allows to stand overnight and filtered. The pH of the filtrate is adjusted by sulfuric acid to pH 5-6, allows to stand in ice bath overnight and filtered. The filtrate is then loaded and exchanged on the cation and anion mixed resin, and the exchange fluid is concentrated to a certain volume. Ethanol is added to precipitate, allows to stand in ice bath overnight and filtered. After volatilization of ethanol from the precipitate, and concentrated to give the *Radix Angelicae Sinensis* extract.

The method of extracting *Radix Angelicae Sinensis* by distillation: the powder of *Radix Angelicae Sinensis* is weighed and added into flask, extracted with distilled water by reflux. Under certain conditions, the materials and extract are distilled under reduced pressure to give distillate. Proper amount of NaCl is added and allows to stand to separate into two layers; separating the volatile oils of *Radix Angelicae Sinensis* with separating funnel, and the remainder is concentrated to obtain the *Radix Angelicae Sinensis* extract.

The method of extracting *Rhizoma Chuanxiong* with ethanol and resin: *Rhizoma Chuanxiong* is taken, pulverized and added into Soxhlet extractor, refluxed and extracted by ethanol, filtered. The filtrate is concentrated under reduced pressure to dryness. Proper amount of water is added to dissolve the solid by heating and filtered. The filtrate is loaded onto the pre-activated macroporous resin column, eluted with water firstly, discarding the eluent, and then eluted with ethanol, the eluents are combined and concentrated by recovering ethanol, and further concentrated to give the *Rhizoma Chuanxiong* extract.

The method of extracting *Rhizoma Chuanxiong* with ethanol: the medicinal material of *Rhizoma Chuanxiong* is taken, pulverized to some extent, and extracted with ethanol for several times. The extracts are combined and filtered; ethanol is recovered to give the *Rhizoma Chuanxiong* extract.

In addition to extracting the Chinese medicines alone, *Radix Angelicae Sinensis, Rhizoma Chuanxiong* and *Radix Paeoniae alba* may be extracted in combinations, for example, the mixture of pulverized *Radix Angelicae Sinensis, Rhizoma Chuanxiong* and *Radix Paeoniae alba* is extracted with boiling water and filtered. The filtrates are combined and concentrated appropriately, into which ethanol is added to precipitate, allow to stand and the supernatant is concentrated by recovering ethanol to give the extractum of the medicinal materials.

Particularly, the invention provides processes for preparation of the composition, the processes comprising the following steps:

a. Providing medicinal materials according to any one of the proportions of the medicinal materials mentioned-above;

b. *Radix Angelicae Sinensis, Rhizoma Chuanxiong, Radix Paeoniae alba* and *Rhizoma Corydalis* is extracted by refluxing with ethanol respectively, the extracts are combined and stored for use; or adding ethanol into a mixture of *Radix Angelicae Sinensis, Rhizoma Chuanxiong, Radix Paeoniae alba* and *Rhizoma Corydalis,* extracting by refluxing with heating, and the reflux solution is stored for use;

c. The residues of step (b) and other five medicinal materials except *Ramulus Uncariae cum Unci* are decocted with water for several times, adding *Ramulus Uncariae cum Unci* in the last decoction; the decoctums are combined and concentrated;

d. The concentrated decoctum of step (c) is precipitated by addition of ethanol, the obtained supernatant and the above-obtained reflux solution are combined, concentrated by recovering ethanol to give the composition in the extractum form;

e. Mixing the extracts of step (d) with appropriate adjuvants to obtain desired formulations.

Preferably, in step (b), ethanol is added into a mixture of *Radix Angelicae Sinensis, Rhizoma Chuanxiong, Radix Paeoniae alba* and *Rhizoma Corydalis* to extract under reflux by heating and the reflux solution is stored for use. The residues and other five medicinal materials except *Ramulus Uncariae cum Uncis* are decocted with water for three times; at the third decoction, *Ramulus Uncariae cum Uncis* is added to decoct. The decoctums are combined and concentrated until the relative density of extract is about 1.09-1.13 at 55°C, and precipitated by adding ethanol. The obtained supernatant and the reflux solution are combined, and ethanol is recovered to give the extract of the medicinal materials.

In the above process, the concentration of ethanol in the step (b) is about 60%-80% (v/v), and preferably 70% (v/v); and the time for each reflux is 0.5-1.5 hours, and preferably about 1.0 hour; each decoction time of the residues and other medicinal materials is 0.5-1.5 hours, and preferably about 1.0 hour; the relative density of extract after concentration is preferably about 1.10 at 55°C; in step (c), preferably, the weight of water is 5 times of that of the medicinal materials when decocting; in step (d), when precipitating by addition of ethanol, the final content of ethanol is about 60%-70%(v/v), and preferably 65% (v/v); the relative density of the concentrated extractum is about 1.15 at 55°C.

The extract in the extractum form obtained according to the above-mentioned process may be dried by common processs in the prior art to obtain dried extractum or extractum powder, and the spray-dried process is selected with the inlet heat air temperature at about 165-205°C, preferably about 185°C.

The process of the invention also comprises the step of producing desired pharmaceutical formulations by mixing the obtained extractum, dry extractum or extractum powder and pharmaceutically acceptable carries, the preferable formulations are pills, and the more preferable are coating pills, and the most preferable are stomach-soluble film-coating pills.

Therefore, the process of the invention further comprises coating step. The coating step may be the film-coating process or ordinary coating process which are known in the prior art, and stomach-soluble film-coating process is preferred.

Especially, the film-coating materials can use stomach-soluble coating materials, for example, using Opadry® produced by Shanghai COLORCON Coating Technology Co. Ltd as the coating materials. The coating conditions are as follows: water is selected as solvent, the concentration of coating solution is 14-20%(w/w), preferably 18%(w/w); the increase in the pill weight after coating is 2%-6%, preferably 4%; inlet air temperature is at 40-90°C, preferably at 65°C; the temperature of tablet bed is at 40-70°C, preferably at 43°C; the spraying pressure is at 1.5-2.5 bar, preferably 2.0bar; the rotation speed of the coating vessel is at 15-20rpm, preferably at 17rpm; inlet flow rate is at 2-4g/min, preferably at 3g/min.

On the other hand, the common coating comprises the steps of adding appropriate amount of talc powder and colorant, and then polishing with wax. The detailed conditions are as follows: an appropriate amount of 1% talc powder is taken and diluted with water to wet the blank pills. Up to the time when the talc powder spread well, 1% of colorant is added. When the colorant spread well, 10% of wax is added to polish. The rotation speed of the coating vessel is at 5-30rpm.

In the process of the invention, the steps of extracting with ethanol, decocting with water and precipitating with ethanol are carried out according to the conventional amount of the solvents and the processs recorded in the Chinese Pharmacopoeia. The production rate of extractum, i.e. on the basis of the dry weight of the medicinal materials, the weight percentage of the obtained dried extractum is usually about 16%.

As calculated based on the raw medicine amount, the adult dosage of the concentrated pills of this invention is equal to 12-20g raw medicine, preferably 15-17g. The administration times depends on the state of an illness, the administration times is commonly 2-3 times/day.

The composition of this invention provides a more reasonable and safer recipe. Compared with the prior art (Chinese Patent No. ZL 93100050.5, hereinafter referred as "YXQN-01"), the invention removes *Herba* Asari, which is prone to producing toxicity, from the recipe, and makes the recipe more reasonable and safer without reducing curative effects of treating headache.

The formulation in Chinese Patent No. ZL93100050.5 is granule, which is a new dosage form developed from Chongji. The recent decade witnesses its rapid progress. Not only does the granule keep the Chongji's features of convenient administration, preservation and transportation, but also overcomes Chongji's defects of high-content of sugar, making it suitable for the old and diabetics. On the other hand, due to the decrease in the amount of the adjuvants in the granule, the size thereof is much smaller, and the active ingredient therein is higher than that of the Chongj. However, the granule contains more extractum and much less additives, thus makes the granule easily absorb dampness, conglomerate and change its color etc. Meanwhile, the granule is extreme bitterness in taste and some may either produce strong stimulating effects on stomach or tend to be damaged in stomach. All these problems of the granule need to be solved.

The preferred embodiment of the invention is as follows: the extractum is spray-dried to obtain the concentrated pills, and combines some other processes such as film-coating to give a new dosage form. In practice, it can overcome the problems of the known granules in the prior art. Determination of the dissolution rate showed that the dissolution curve of the concentrated pills (CP) of this invention (hereinafter referred as "Yang Xue Qing Nao Pill") is similar to that of the Yang Xue Qing Nao Granule, indicating that the dissolution *in vitro* of Yang Xue Qing Nao CP is identical with that of the Yang Xue Qing Nao Granule.

The significant inhibitory effects of the two compositions on the writhing reaction in mice caused by acetic acid are investigated to further demonstrate the excellent effect of the composition of the invention and verify the inventive step of the invention.

**The effect of the Chinese medicinal composition on pain relief and anti-hypoxia in mice**

1. Aim

The experiment is carried out to investigate the effect of two compositions, the present composition YXQN-02 and YXQN-01 in the prior art, on pain relief and anti-hypoxia in mice.

2. Materials

2.1 Drugs and their preparation

2.1.1 Tested drugs

YXQN-01, the drug of Chinese Patent No. ZL 93100050.5, is prepared by Research Institute of Tianjin Tasly Group with batch number of 20041201. Concerning the preparation method, please refer to the comparison example. Before experiment, the drug is diluted with distilled water to obtain the solution of 1.4g raw medicine/ml, 0.7g raw medicine/ml and 0.35g raw medicine/ml for intragastric administration in mice.
YXQN-02 is prepared by Research Institute of Tianjin Tasly Group with batch number of 20041201 according to the method of Example 5. Before experiment, the drug is diluted with distilled water to obtain the solution of 1.4g raw medicine/ml, 0.7g raw medicine/ml and 0.35g raw medicine/ml for intragastric administration in mice.

2.1.2 Positive control drugs

Aspirin enteric-soluble tablet (25mg/tablet) is produced by Shijiazhuang Kangli Pharmaceutical Co. Ltd. with the batch number of 041219. Before experiment, the drug is diluted with 0.5% CMC-Na to give a suspension of 10mg/ml for intragastric administration in mice.
Propranolol (7.0mg/tablet) is produced by Tianjin Lisheng Pharmaceutical Co. Ltd. with the batch number of 0411015. Before experiment, the drug is diluted with distilled water to give the solution of 5mg/ml for intragastric administration in mice.

2.2 Reagents

Glacial acetic acid: Tianjin No. 1 Chemistry Factory, batch number: 890508.
Vaseline: Tianjin Petroleum Chemistry Experimental Factory, batch number: 860404.
Soda-lime: Shanghai Nahui Desiccation Reagent Factory, batch number: 20040724.

2.3 Apparatus

AE-200 Electronic Analytical Balance (Mettler Toledo Instruments (Shanghai) Co. Ltd);
TF2-thermal radiation pain threshold detector (lamp-house 12V/50W), produced by Institute of Materia Medica, Chinese Academy of Medical Sciences & Peking Union Medical College;
Stopwatch; 250ml wide-mouth milled bottles; and scissors.

2.4 Animals

ICR mice, male and female in half, weighing 20±2g (certificate No: SCXK (Beijing) 2002-0003) are provided by Beijing Weitonglihua Experimental Animal Technology Co., Ltd. (Beijing, P.R. China)

3. Statistical analysis

SPSS10.0 software package is used to carry out the statistical analysis. All the data are expressed as ($x \pm s$) and the $t$-test is employed in inter-group comparison.

4. Methods and results

4.1 Effect of YXQN-01 and YXQN-02 on pains caused by acetic acid in mice

4.1.1 Methods

112 of the healthy mice, male and female in half, are randomly divided into eight groups according to body weight and sex, 14 mice in one group: the control group, high, middle and low dosage groups of YXQN-01 (28, 14 and 7g raw medicine/kg, respectively); high, middle and low dosage groups of YXQN-02 (28, 14 and 7g raw medicine/kg, respectively) and aspirin enteric-soluble tablets group (200mg/kg) as the positive control. Both YXQN-01 and YXQN-02 are administered intragastrically at a dosage of 0.2ml/10g body weight, one time per day and continues for five days. The aspirin enteric-soluble tablets are administered only once. The control group is administered with equal-volume distilled water. One hour after the last administration, each mouse is injected with 0.6% acetic acid intraabdominally at a dosage of 0.1ml/10g. Acetic acid-elicited writhing frequency is recorded, and calculates the inhibitory rate of the drugs to writhing reactions to evaluate the effect of pain relief. The equation is as follows:

$$\text{Inhibitory rate}(\%) = \frac{\text{writhing frequency of the control group - writhing frequency of the adminstered group}}{\text{writhing frequency of the control group}} \times 100\%$$

4.1.2 Results

After administering the tested drugs intragastrically for consecutive five days, the inhibitory rate of YXQN-01 at dosage of 28, 14 and 7g raw medicine/kg is 49.88% (P<0.001), 45.99% (P<0.01) and 33.58% (P<0.05), respectively; and the inhibitory rate of YXQN-02 at 28, 14 and 7g raw medicine/kg is 44.53% (P<0.01), 62.29% (P<0.001) and 43.07% (P<0.05), respectively. As the result shown, both YXQN-01 and YXQN-02 have the significant inhibitory effect on the writhing reactions caused by acetic acid. Compared with the control group, the inhibitory rate of the aspirin enteric-soluble tablets group (0.2g/kg) is 69.83% (P<0.001), and also have the significant inhibitory effect. The results are listed in Table 1.

Table 1 Effect of YXQN-01 and YXQN-02 on pain caused by acetic acid in mice ($x\pm s$)

| Groups | Dosage (g/kg) | Number of mice | Writhing times in 15min | Inhibitory rate(%) |
|---|---|---|---|---|
| Control group | - | 14 | 29.36 $\pm$ 7.78 | - |
| YXQN-01 | 28 | 14 | 16.50 $\pm$ 7.56*** | 49.88 |
| YXQN-01 | 14 | 14 | 18.93 $\pm$ 6.79** | 45.99 |
| YXQN-01 | 7 | 14 | 22.36 $\pm$ 9.48* | 33.58 |
| YXQN-02 | 28 | 14 | 17.71 $\pm$ 9.56** | 44.53 |
| YXQN-02 | 14 | 14 | 13.93 $\pm$ 7.14*** | 62.29 |
| YXQN-02 | 7 | 14 | 21.71 $\pm$ 10.03* | 43.07 |
| Aspirin enteric-soluble tablets group | 0.2 | 14 | 8.57 $\pm$ 4.85*** | 69.83 |

Note: *P<0.05 , **P<0.01, ***P<0.001, compared with the control group.

**4.2 Effect of YXQN-01 and YXQN-02 on tail-flick induced by light radiant heat in mice**

4.2.1 Methods

4.2.1.1 Principle of experiment

TF2- thermal radiation pain threshold detector uses the 12V/50W mini focus lamp. The lamp-house may produce light with certain intensity that is allowed to focus on mice's tail by lens to cause pain.

4.2.1.2 Selection for qualified mice

Healthy and comparable-body weight mice are used, the tail-flick latency (TFL) from simulating the tail of the mice with thermal radiation to tail-flick reaction is determined as pain threshold. The mice with the pain threshold of more than 2s and less than 5s are used in the experiment, the determined pain thresholds are regarded as the basic pain thresholds.

4.2.1.3 Animal Grouping and administration

112 of the qualified mice are randomly divided into eight groups according to sex and pain threshold, 14 mice in one group: the control group, high, middle and low dosage group of YXQN-01 (28, 14 and 7g raw medicine/kg, respectively); high, middle and low dosage group of YXQN-02 (28, 14 and 7g raw medicine/kg, respectively) and aspirin enteric-soluble tablets group (200mg/kg) as the positive control. Both YXQN-01 and YXQN-02 are administered intragastrically at a dosage of 0.2ml/10g body weight, one time per day and continues for five days. The aspirin enteric-soluble tablets are administered only once. The control group is administered with equal-volume distilled water. One hour after the last administration, the mouse is placed into the fixed barrel of TF2-thermal radiation pain threshold detector, leaving the tail outside (the tail is cleaned with 75% ethanol before experiment). The inside skin at 1.5 cm to the tip of tail is aimed to the focus of the lens. After the mice calm down, the pain inducing experiment is started. In order to prevent the skin from being scalded, lighting time is limited to 16s. The pain threshold-enhancing rate is calculated as follows to access the pain relief effect of the tested drugs;

$$\text{The pain threshold enhancing rate (\%)} = \frac{\text{TFL after administration} - \text{basic TFL}}{\text{basic TFL}} \times 100\%$$

4.2.2 Results

After administering intragastrically the tested drugs for consecutive five days, the pain threshold-enhancing rate of YXQN-01 at dosage of 28 and 14g raw medicine/kg is 100.98% (P<0.01) and 130.30% (P<0.01), respectively; and the drug of the invention YXQN-02 at 28g raw medicine/kg is 160.70% (P<0.05), respectively. As the result shown, compared with the control group, both the YXQN-01 group and YXQN-02 group have significant difference. The pain threshold-enhancing rate of the aspirin enteric-soluble tablets group (0.2g/kg) is 176.89% (P<0.01), compared with the control group, it also has significant difference. Results are listed in Table 2.

Table 2 Effect of YXQN-01 and YXQN-02 on TFL in mice ($x \pm s$)

| Groups | Dosage (g/kg) | Number of mice | Basic pain Threshold (s) | Pain threshold after administration (s) | Enhancing rate (%) |
|---|---|---|---|---|---|
| Control group | - | 14 | 2.89+0.78 | 3.42 ± 0.82 | - |
| YXQN-01 | 28 | 14 | 2.93 ± 0.66 | 5.89 ± 2.42** | 100.98 |
| YXQN-01 | 14 | 14 | 2.90 ± 0.67 | 6.68 ± 3.74** | 130.30 |
| YXQN-01 | 7 | 14 | 2.98 ± 0.75 | 4.89 ± 3.32 | 64.03 |
| YXQN-02 | 28 | 14 | 2.87 ± 0.72 | 7.49 ± 4.31** | 160.70 |
| YXQN-02 | 14 | 14 | 2.88 ± 0.72 | 4.67 ± 3.03 | 62.28 |
| YXQN-02 | 7 | 14 | 2.99 ± 0.76 | 4.76 ± 2.84 | 59.57 |
| aspirin enteric- | 0.2 | 14 | 2.94 ± 0.77 | 8.13 ± 4.35** | 176.89 |

(continued)

| Groups | Dosage (g/kg) | Number of mice | Basic pain Threshold (s) | Pain threshold after administration (s) | Enhancing rate (%) |
|---|---|---|---|---|---|
| soluble tablets | | | | | |

Note: *P<0.05, **P<0.01, compared with the control group.

5. Conclusion

Both YXQN-01 and YXQN-02 can significantly reduce the acetic acid-caused writhing frequency, and prolong the TFL caused by lighting radiation, demonstrating the substantially identical pain relief effect.

**Comparison of the dissolution rate for the pills of the invention and the known granules in the prior art**

The experiment is carried out in accordance with the determination method of dissolution recorded in the Appendices X C of Chinese Pharmacopoeia (Edition 2000, Volume II).
Rotating basket method is used as the determination method, in which the dissolution media is 900ml of degassed water at 37±0.5°C, and the rotating speed is 100 r/min.

1. Determination of the dissolution

4g of Yang Xue Qing Nao granule (prepared by a method of the comparison example) and 2.5g of Yang Xue Qing Nao Pill (prepared by a method of Example 5) are weighed and added into rotating baskets, respectively. Six hours after turning on electromotor, sample, filter immediately and the successive filtrate are collected. 10μl of the successive filtrate is accurately measured and respectively injected into HPLC, and determine.

2. Determination of the dissolution rate

Four samples of 4g Yang Xue Qing Nao granule is weighed and added into four baskets, and marked as "Sample 1", "Sample 2", "Sample 3" and "Sample 4", respectively. Turning on electromotor immediately, the samples are sampled at the predetermined time at 5, 10, 25, 40, 60, 80 and 100min after turning on the electromotor. 5ml is sampled and then 5ml of water at 37°C is added immediately. The sample is filtered immediately and the successive filtrate is collected, 10μl of which is measured accurately and injected into HPLC, and determine. Four samples of 2.5g Yang Xue Qing Nao Pill is weighed and added into four baskets, and marked as "Sample 1", "Sample 2", "Sample 3" and "Sample 4", respectively. Turning on electromotor immediately, the samples are sampled at the predetermined time at 5, 10, 25, 40, 60, 80 and 100min after turning on the electromotor. The following steps are identical with that of the Granule. According to the results, the cumulative release percentages of the Granule and Pill are calculated (See Table 3 and Table 4).

Table 3 Cumulative release percentage of Yang Xue Qing Nao Granule (%)

| T(min) | 5 | 10 | 25 | 40 | 60 | 80 | 100 |
|---|---|---|---|---|---|---|---|
| Sample 1 | 14.78 | 25.49 | 44.60 | 54.56 | 66.46 | 79.24 | 84.71 |
| Sample 2 | 15.76 | 25.68 | 45.06 | 56.19 | 69.44 | 80.64 | 88.27 |
| Sample 3 | 14.34 | 25.27 | 43.39 | 53.08 | 65.27 | 78.62 | 86.98 |
| Sample 4 | 15.54 | 25.68 | 45.32 | 53.79 | 67.68 | 79.58 | 97.39 |
| Average | 15.10 | 25.53 | 44.59 | 54.40 | 67.21 | 79.52 | 89.34 |

Table 4 Cumulative release percentage of Yang Xue Qing Nao Pill (%)

| T(min) | 10 | 25 | 40 | 60 | 80 | 100 | 120 |
|---|---|---|---|---|---|---|---|
| Sample 1 | 27.03 | 44.07 | 58.08 | 73.59 | 78.88 | 85.54 | 95.85 |

(continued)

| T(min) | 10 | 25 | 40 | 60 | 80 | 100 | 120 |
|---|---|---|---|---|---|---|---|
| Sample 2 | 26.05 | 45.73 | 58.57 | 72.20 | 74.05 | 76.71 | 82.75 |
| Sample 3 | 27.71 | 41.90 | 59.13 | 73.34 | 80.98 | 82.56 | 90.93 |
| Sample 4 | 25.06 | 44.25 | 64.56 | 70.32 | 76.19 | 77.46 | 85.38 |
| Average | 26.46 | 43.99 | 60.08 | 72.36 | 77.53 | 80.57 | 88.73 |

3. Statistic analysis

3.1 Calculation of $T_{50}$ and $T_d$

By taking Int. $\ln[-\ln(1-F)]$ as the variable, the regression equations of the Granule and Pill are set up and got the dissolution parameter $T_{50}$ and $T_d$. As shown in the results, the dissolution *in vitro* of Yang Xue Qing Nao Pill is substantially identical with that of the Granule.

Table 5 Comparison of the dissolution parameters between the Granule and Pill

| Formulations | $T_{50}$(min) | $T_d$(min) |
|---|---|---|
| Yang Xue Qing Nao Pill of the invention | 29.16 | 46.84 |
| Yang Xue Qing Nao Granule | 29.66 | 49.81 |

3.2 Calculation of similarity factor ($f_2$)

The cumulative release percentages at 10, 25, 40, 60, 80 and 100 min of the two drugs are used in similarity factor equation and the result is as follows:

$$f_2 = 50 \times \log\{[1+(1/n)\sum_{t=1}^{n}(R_t-S_t)^2]^{-0.5} \times 100\} = 68.77 \; > 50$$

According to the *Guidance for industry bioavailability and bioequivalence studies for orally administered drug products* issued by U.S. Food and Drug Administration (FDA), the similarity factor>50 shows that both have the similar dissolution curve, indicating that Yang Xue Qing Nao Granule and Yang Xue Qing Nao Pill have identical dissolution *in vitro*.

**Description of drawings**

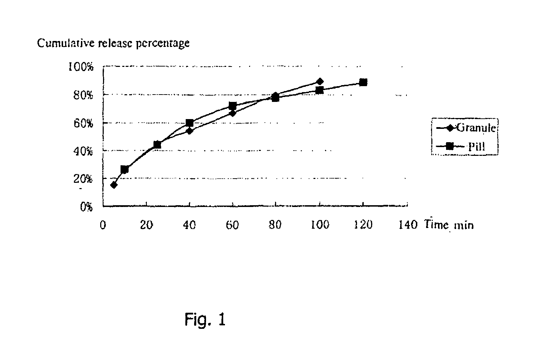

Figure 1 is the cumulative release percentage-time curves of Yang Xue Qing Nao Pill of the invention and Yang Xue Qing Nao Granule.

**Best mode for carrying out the invention**

Hereinafter, the invention will be described in more detail with reference to Examples, but the invention is not limited to these Examples.

**Examples**

Comparison example: Preparation of the known YXQN-01 granule

The example is carried out according to the method of Chinese Patent No. ZL 93100050.5.

1. Extraction of the Chinese medicines

The following medicinal materials are taken: 405.6 g of *Radix Angelicae Sinensis,* 405.6 g of *Rhizoma Chuanxiong,* 324.3 g of *Radix Paeoniae alba,* 324.3 g of *Radix Rehmanniae Preparata,* 810.8 g of *Ramulus Uncariae cum Uncis,* 810.8 g of *Caulis Spatholobi,* 810.8 g of *Spica Prunellae,* 810.8 g of *Semen Cassiae,* 810.8 g of *Concha Mar-*

*garitifera Usta,* 405.6 g of *Rhizoma Corydalis* and 80.8 g of *Herba Asari.*

In the above-mentioned eleven medicinal materials, *Radix Angelicae Sinensis, Rhizoma Chuanxiong, Radix Paeoniae alba* and *Rhizoma Corydalis* are mixed with 70% ethanol and refluxed for 1 hour by heating, the reflux solution is stored for use. The residues are extracted with other six Chinese medicines such as *Radix Rehmanniae Preparata* etc. except *Ramulus Uncariae cum Uncis* for three times, one hour for each. At the third decoction, the *Ramulus Uncariae cum Uncis* is added. The decoctions are combined, concentrated under reduced pressure till the relative density is 1.10 at 55°C. Ethanol is added to the obtained residue to make the final concentration of ethanol is 65%, allow to stand for 24 hours, filtered the precipitate and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at about 185°C.

2. Preparation of granules

The fine powder obtained above is mixed with dextrin to make into granules by spray-drying method.

Example 1 Preparation of granules of the Chinese medicinal compositions of the invention

1. Extraction of the Chinese medicines

The following medicinal materials are taken: 68.5 g of *Radix Angelicae Sinensis,* 68.5 g of *Rhizoma Chuanxiong,* 55 g of *Radix Paeoniae alba,* 55 g of *Radix Rehmanniae Preparata,* 136.9 g of *Ramulus Uncariae cum Uncis,* 136.9 g of *Caulis Spatholobi,* 136.9 g of *Spica Prunellae,* 136.9 g of *Semen Cassiae,* 136.9 g of *Concha Margaritifera Usta* and 68.5 g of *Rhizoma Corydalis.*

In the above-mentioned ten medicinal materials, *Radix Angelicae Sinensis, Rhizoma Chuanxiong, Radix Paeoniae alba* and *Rhizoma Corydalis* are mixed with 70% ethanol and refluxed for 1 hour by heating, the reflux solution is stored for use. The residues are extracted with other five Chinese medicines such as *Radix Rehmanniae Preparata* etc. except Romulus *Uncariae cum Uncis* for three times, one hour for each. At the third decoction, the *Ramulus Uncariae cum Uncis* is added. The decoctions are combined, concentrated under reduced pressure till the relative density is 1.10 at 55°C. Ethanol is added to the obtained residue to make the final concentration of ethanol is 65%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 185°C.

2. Preparation of granules

The fine powder obtained above is mixed with dextrin to make into granules by spray-drying method.

Example 2 Preparation of tablets of the Chinese medicinal compositions of the invention

1. Extract of Chinese medicines

The following medicinal materials are taken: 68.5 g of *Radix Angelica Sinensis,* 68.5 g of *Rhizoma Chuanxiong,* 55 g of *Radix Paeoniae alba,* 55 g of *Radix Rehmanniae Preparata,* 136.9 g of *Ramulus Uncariae cum Uncis,* 136.9 g of *Caulis Spatholobi,* 136.9 g of *Spica Prunellae,* 136.9 g of *Semen Cassiae,* 136.9 g of *Concha Margaritifera Usta* and 68.5 g of *Rhizoma Corydalis.*

In the above-mentioned ten medicinal materials, *Radix Angelicae Sinensis, Rhizoma Chuanxiong, Radix Paeoniae alba* and *Rhizoma Corydalis* are added mixed 70% ethanol and refluxed for 1 hour by heating, the reflux solution is stored for use. The residues are extracted with other five Chinese medicines such as Radix Rehmanniae Preparata etc. except *Ramulus Uncariae cum Uncis* for three times, one hour for each. At the third decoction, *Ramulus Uncariae cum Uncis* is added. The decoctions are combined, concentrated under reduced pressure till the relative density is 1.10 at 55°C. Ethanol is added to the obtained residue to make the final concentration of ethanol is 65%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 185°C.

2. Preparation of tablets

The fine powder obtained above is mixed with starch to press into tablets.

Example 3 Preparation of capsules of the Chinese medicinal compositions of the invention

1. Extract of Chinese medicines

The following medicinal materials are taken: 68.5 g of *Radix Angelicae Sinensis,* 68.5 g of *Rhizoma Chuanxiong,* 55 g of *Radix Paeoniae alba,* 55 g of *Radix Rehmanniae Preparata,* 136.9 g of *Ramulus Uncariae cum Uncis,* 136.9 g of *Caulis Spatholobi,* 136.9 g of *Spica Prunellae,* 136.9 g of *Semen Cassiae,* 136.9 g of *Concha Margaritifera Usta* and 68.5 g of *Rhizoma Corydalis.*

In the above-mentioned ten medicinal materials, *Radix Angelicae Sinensis, Rhizoma Chuanxiong, Radix Paeoniae alba* and *Rhizoma Corydalis* are mixed with 70% ethanol and refluxed for 1 hour by heating, the reflux solution is stored for use. The residues are extracted with other five Chinese medicines such as Radix Rehmanniae Preparata etc. except *Ramulus Uncariae cum Uncis* for three times, one hour for each. At the third decoction, the *Ramulus Uncariae cum Uncis* is added. The decoctions are combined, concentrated under reduced pressure till the relative density is 1.10 at 55°C. Ethanol is added to the obtained residue to make the final concentration of ethanol is 65%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 185°C.

2. Preparation of capsules

The fine powder obtained above is mixed with starch and loaded into No.1 capsules.

Example 4 Preparation of coating pills of the Chinese medicinal compositions of the invention

1. Extraction of the Chinese medicines

The following medicinal materials are taken: 405.6 g of *Radix Angelicae Sinensis,* 405.6 g of *Rhizoma Chuanxiong,* 324.3 g of *Radix Paeoniae alba,* 324.3 g of *Radix Rehmanniae Preparata,* 810.8 g of *Ramulus Uncariae cum Uncis,* 810.8 g of *Caulis Spatholobi,* 810.8 g of *Spica Prunellae,* 810.8 g of *Semen Cassiae,* 810.8 g of *Concha Margaritifera Usta,* 405.6 g of *Rhizoma Corydalis.*

In the above-mentioned ten medicinal materials, *Radix Angelicae Sinensis, Rhizoma Chuanxiong, Radix Paeoniae alba* and *Rhizoma Corydalis* are mixed with 70% ethanol and refluxed for 1 hour by heating, the reflux solution is stored for use. The residues are extracted with other five Chinese medicines such as *Radix Rehmanniae Preparata* etc. except *Ramulus Uncariae cum Uncis* for three times, one hour for each. At the third decoction, the *Ramulus Uncariae cum Uncis* is added. The decoctions are combined, concentrated under reduced pressure till the relative density is 1.10 at 55°C. Ethanol is added to the obtained residue to make the final concentration of ethanol is 65%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 185°C.

2. Preparation of the concentrated pills

The obtained extractum powder is mixed with microcrystaline cellulose, and the amount of microcrystaline cellulose is 35% of the weight of the extractum powder. Water is added as binding agent to produce the blank pills.

3. Coating the pills

The resultant blank pills are coated with stomach-soluble Opadry® produced by Shanghai COLORCON Coating Technology Co. Ltd. as the coating materials to give the stomach-soluble coating concentrated pills of the invention.

The coating conditions are as follows: water is selected as solvent, the concentration of coating solution is 18% (w/w), the increase in weight of the pill after coating is 4%, the inlet air temperature is at 65°C, the temperature of tablet bed is at 43°C, the spraying pressure is at 2.0bar, the rotation speed of the coating vessel is at 17rpm and the inlet flow rate is at 3g/min.

Example 5 Preparation of coating pills of the Chinese medicinal compositions of the invention

1. Extraction of the Chinese medicines

The following medicinal materials are taken: 405.6 g of *Radix Angelicae Sinensis,* 405.6 g of *Rhizoma Chuanxiong,* 324.3 g of *Radix Paeoniae alba,* 324.3 g of *Radix Rehmanniae Preparata,* 810.8 g of *Ramulus Uncariae cum Uncis,* 810.8 g of *Caulis Spatholobi,* 810.8 g of *Spica Prunellae,* 810.8 g of *Semen Cassiae,* 810.8 g of *Concha Margaritifera Usta,* 405.6 g of *Rhizoma Corydalis.*

In the above-mentioned ten medicinal materials, *Radix Angelicae Sinensis, Rhizoma Chuanxiong, Radix* Paeoniae alba and *Rhizoma Corydalis* are mixed with 60% ethanol and refluxed for 0.5 hours by heating, the reflux solution is stored for use. The residues are extracted with other five Chinese medicines such as *Radix Rehmanniae Preparata* etc. except *Ramulus Uncariae cum Uncis* for three times, 0.5 hours for each. At the third decoction, the *Ramulus Uncariae cum Uncis* is added. The decoctions are combined, concentrated under reduced pressure till the relative density is 1.10 at 55°C. Ethanol is added to the obtained residue to make the final concentration of ethanol is 60%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 165°C.

2. Preparation of the concentrated pills

The obtained extractum powder is mixed with dextrin, and the amount of dextrin is 25% of the weight of the extractum powder. Water is added as binding agent to produce the blank pills.

3. Coating the concentrated pills

The resultant pills are coated with stomach-soluble Opadry® produced by Shanghai COLORCON Coating Technology Co. Ltd. as the coating materials to give the stomach-soluble coating concentrated pills of the invention.

The coating conditions are as follows: water is selected as solvent, the concentration of coating solution is 14% (w/w), the increase in weight of the pill after coating is 2%, the inlet air temperature is at 85°C, the temperature of tablet bed is at 40°C, the spraying pressure is at 2.0bar, the rotation speed of the coating vessel is at 17rpm and the inlet flow rate is at 3g/min.

Example 6 Preparation of coating pills of the Chinese medicinal compositions of the invention

1. Extraction of the Chinese medicines

The following medicinal materials are taken: 405.6 g of *Radix Angelicae Sinensis,* 405.6 g of *Rhizoma Chuanxiong,* 324.3 g of *Radix Paeoniae alba,* 324.3 g of *Radix Rehmanniae Preparata,* 810.8 g of *Romulus Uncariae cum Uncis,* 810.8 g of *Caulis Spatholobi,* 810.8 g of *Spica Prunellae,* 810.8 g of *Semen Cassiae,* 810.8 g of *Concha Margaritifera Usta,* 405.6 g of *Rhizoma Corydalis.*

In the above-mentioned ten medicinal materials, *Radix Angelicae Sinensis, Rhizoma Chuanxiong, Radix Paeoniae alba* and *Rhizoma Corydalis* are mixed with 80% ethanol and refluxed for 1.5 hours by heating, the reflux solution is stored for use. The residues are extracted with other five Chinese medicines such as *Radix Rehmanniae Preparata* etc. except *Ramulus Uncariae cum Uncis* for three times, 1.5 hours for each. At the third decoction, the *Ramulus Uncariae cum Uncis* is added. The decoctions are combined, concentrated under reduced pressure till the relative density is 1.10 at 55°C. Ethanol is added to the obtained residue to make the final concentration of ethanol is 70%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 205°C.

2. Preparation of the concentrated pills

The obtained powders are mixed with microcrystaline cellulose, and the amount of microcrystaline cellulose is 25% of the weight of the extractum powder. Water is added as binding agent to produce the blank pills.

3. Coating the concentrated pills

The resultant pills are coated with stomach-soluble opadry® produced by Shanghai COLORCON Coating Technology Co. Ltd. as the coating materials to give the stomach-soluble coating concentrated pills of the invention.

The coating conditions are as follows: water is selected as solvent, the concentration of coating solution is 20% (w/w), the increase in weight of the pill after coating is 6%, the inlet air temperature is at 90°C, the temperature of tablet bed is at 45°C, the spraying pressure is at 2.50bar, the rotation speed of the coating vessel is at 20rpm and the inlet flow rate is at 4g/min.

Example 7 Preparation of coating pills of the Chinese medicinal compositions of the invention

1. Extraction of the Chinese medicines

The following medicinal materials are taken: 240.1 g of *Radix Angelicae Sinensis,* 240.1 g of *Rhizoma Chuanxiong,* 120.0 g of *Radix Paeoniae alba,* 120.0 g of *Radix Rehmanniae Preparata,* 600.0 g of *Ramulus Uncariae cum Uncis,* 600.0 g of *Caulis Spatholobi,* 600.0 g of *Spica Prunellae,* 600.0 g of *Semen Cassiae,* 600.0 g of *Concha Margaritifera Usta,* 240.0 g of *Rhizoma Corydalis.*

In the above-mentioned ten medicinal materials, *Radix Angelicae Sinensis, Rhizoma Chuanxiong, Radix Paeoniae alba and Rhizoma Corydalis* are mixed with 70% ethanol and refluxed for 1 hour by heating, the reflux solution is stored for use. The residues are extracted with other five Chinese medicines such as Radix *Rehmanniae Preparata* etc. except *Ramulus Uncariae cum Uncis* for three times, one hour for each. At the third decoction, the *Ramulus Uncariae cum Uncis* is added. The decoctions are combined, concentrated under reduced pressure till the relative density is 1.10 at 55°C. Ethanol is added to the obtained residue to make the final concentration of ethanol is 65%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 185°C.

2. Preparation of the concentrated pills

The obtained powders are mixed with microcrystaline cellulose, and the amount of microcrystaline cellulose is 35% of the weight of the extractum powder. Water is added as binding agent to produce the blank pills.

3. Coating the concentrated pills

The resultant pills are coated with stomach-soluble Opadry® produced by Shanghai COLORCON Coating Technology Co. Ltd. as the coating materials to give the stomach-soluble coating concentrated pills of the invention.

The coating conditions are as follows: water is selected as solvent, the concentration of coating solution is 18% (w/w), the increase in weight of the pill after coating is 4%, the inlet air temperature is at 87°C, the temperature of tablet bed is at 43°C, the spraying pressure is at 2.0bar, the rotation speed of the coating vessel is at 17rpm and the inlet flow rate is at 3g/min.

Example 8 Preparation of coating pills of the Chinese medicinal compositions of the invention

1. Extraction of the Chinese medicines

The following medicinal materials are taken: 540.2 g of *Radix Angelicae Sinensis,* 540.0 g of *Rhizoma Chuanxiong,* 480.0 g of *Radix Paeoniae alba*, 480.0 g of *Radix Rehmanniae Preparata*, 900.0 g of *Ramulus Uncariae cum Uncis,* 900.0 g of *Caulis Spatholobi,* 900.0 g of *Spica Prunellae,* 900.0 g of *Semen Cassiae,* 900.0 g of *Concha Margaritifera Usta,* 540.0 g of *Rhizoma Corydalis.*

In the above-mentioned ten medicinal materials, *Radix Angelicae Sinensis, Rhizoma Chuanxiong, Radix Paeoniae alba* and *Rhizoma Corydalis* are mixed with 70% ethanol and refluxed for 1 hour by heating, the reflux solution is stored for use. The residues are extracted with other five Chinese medicines such as *Radix Rehmanniae Preparata* etc. except *Ramulus Uncariae cum Uncis* for three times, one hour for each. At the third decoction, the *Ramulus Uncariae cum Uncis* is added. The decoctions are combined, concentrated under reduced pressure till the relative density is 1.10 at 55°C. Ethanol is added to the obtained residue to make the final concentration of ethanol is 65%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 185°C.

2. Preparation of the concentrated pills

The obtained powders are mixed with microcrystaline cellulose, and the amount of microcrystaline cellulose is 35% of the weight of the extractum powder. Water is added as binding agent to produce the blank pills.

3. Coating the concentrated pills

The resultant pills are coated with stomach-soluble Opadry® produced by Shanghai COLORCON Coating Technology Co. Ltd. as the coating materials to give the stomach-soluble coating concentrated pills of the invention.

The coating conditions are as follows: water is selected as solvent, the concentration of coating solution is 18% (w/w), the increase in weight of the pill after coating is 4%, the inlet air temperature is at 87°C, the temperature of tablet bed is at 43°C, the spraying pressure is at 2.0bar, the rotation speed of the coating vessel is at 17rpm and the inlet flow rate is at 3g/min.

Example 9 Preparation of coating pills of the Chinese medicinal compositions of the invention

1. Extraction of the Chinese medicines

The following medicinal materials are taken: 240.1 g of *Radix Angelicae Sinensis,* 240.1 g of *Rhizoma Chuanxiong,* 120.0 g of *Radix Paeoniae alba,* 120.0 g of *Radix Rehmanniae Preparata,* 600.0 g of *Ramulus Uncariae cum Uncis,* 600.0 g of *Caulis Spatholobi,* 600.0 g of *Spica Prunellae,* 600.0 g of *Semen Cassiae,* 600.0 g of *Concha Margaritifera Usta,* 240.0 g of *Rhizoma Corydalis.*

In the above-mentioned ten medicinal materials, *Radix Angelicae Sinensis, Rhizoma Chuanxiong, Radix Paeoniae* alba and *Rhizoma Corydalis* are mixed with 60% ethanol and refluxed for 0.5 hours by heating, the reflux solution is stored for use. The residues are extracted with other five Chinese medicines such as *Radix Rehmanniae Preparata* etc. except *Ramulus Uncariae cum Uncis* for three times, 0.5 hours for each. At the third decoction, the *Ramulus Uncariae cum Uncis* is added. The decoctions are combined, concentrated under reduced pressure till the relative density is 1.09 at 55°C. Ethanol is added to the obtained residue to make the final concentration of ethanol is 60%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 165°C.

2. Preparation of the concentrated pills

The obtained powders are mixed with microcrystaline cellulose, and the amount of microcrystaline cellulose is 35% of the weight of the extractum powder. Water is added as binding agent to produce the blank pills.

3. Coating the concentrated pills

The resultant pills are coated with stomach-soluble Opadry® produced by Shanghai COLORCON Coating Technology Co. Ltd. as the coating materials to give the stomach-soluble coating concentrated pills of the invention.

The coating conditions are as follows: water is selected as solvent, the concentration of coating solution is 16% (w/w), the increase in weight of the pill after coating is 2%, the inlet air temperature is at 85°C, the temperature of tablet bed is at 40°C, the spraying pressure is at 1.5bar, the rotation speed of the coating vessel is at 15rpm and the inlet flow rate is at 2g/min.

Example 10 Preparation of coating pills of the Chinese medicinal compositions of the invention

1. Extraction of the Chinese medicines

The following medicinal materials are taken: 540.2 g of *Radix Angelicae Sinensis,* 540.0 g of *Rhizoma Chuanxiong,* 480.0 g of *Radix Paeoniae alba,* 480.0 g of *Radix Rehmanniae Preparata,* 900.0 g of *Ramulus Uncariae cum Uncis,* 900.0 g of *Caulis Spatholobi,* 900.0 g of *Spica Prunellae,* 900.0 g of *Semen Cassiae,* 900.0 g of *Concha Margaritifera Usta,* 540.0 g of *Rhizoma Corydalis.*

In the above-mentioned ten medicinal materials, *Radix Angelicae Sinensis, Rhizoma Chuanxiong, Radix Paeoniae alba* and *Rhizoma Corydalis* are mixed with 70% ethanol and refluxed for 1.5 hours by heating, the reflux solution is stored for use. The residues are extracted with other five Chinese medicines such as *Radix Rehmanniae Preparata* etc. except *Ramulus Uncariae cum Uncis* for three times, 1.5 hours for each. At the third decoction, the *Ramulus Uncariae*

*cum Uncis* is added. The decoctions are combined, concentrated under reduced pressure till the relative density is 1.10 at 55°C. Ethanol is added to the obtained residue to make the final concentration of ethanol is 70%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 205°C.

2. Preparation of the concentrated pills

The obtained powders are mixed with microcrystaline cellulose, and the amount of microcrystaline cellulose is 35% of the weight of the extractum powder. Honey aqueous solution is added as binding agent to produce the blank pills.

3. Coating the concentrated pills

The resultant pills are coated with stomach-soluble opadry® produced by Shanghai COLORCON Coating Technology Co. Ltd. as the coating materials to give the stomach-soluble coating concentrated pills of the invention.
The coating conditions are as follows: water is selected as solvent, the concentration of coating solution is 20% (w/w), the increase in weight of the pill after coating is 6%, the inlet air temperature is at 90°C, the temperature of tablet bed is at 45°C, the spraying pressure is at 2.5bar, the rotation speed of the coating vessel is at 20rpm and the inlet flow rate is at 4g/min.

Example 11 Preparation of coating pills of the Chinese medicinal compositions of the invention

1. Extraction of the Chinese medicines

The following medicinal materials are taken: 405.6 g of *Radix Angelicae Sinensis,* 405.6 g of *Rhizoma Chuanxiong,* 324.3 g of *Radix Paeoniae alba,* 324.3 g of *Radix Rehmanniae Preparata,* 810.8 g of *Ramulus Uncariae cum Uncis,* 810.8 g of *Caulis Spatholobi,* 810.8 g of *Spica Prunellae,* 810.8 g of *Semen Cassiae,* 810.8 g of *Concha Margaritifera Usta,* 405.6 g of *Rhizoma Corydalis.*
In the above-mentioned ten medicinal materials, *Radix Angelicae Sinensis, Rhizoma Chuanxiong, Radix Paeoniae alba* and *Rhizoma Corydalis* are mixed with 80% ethanol and refluxed for 1.5 hours by heating, the reflux solution is stored for use. The residues are extracted with other five Chinese medicines such as *Radix Rehmanniae Preparata* etc. except *Ramulus Uncariae cum Uncis* for three times, 1.5 hours for each. At the third decoction, the *Ramulus Uncariae cum Uncis* is added. The decoctions are combined, concentrated under reduced pressure till the relative density is 1.10 at 55°C. Ethanol is added to the obtained residue to make the final concentration of ethanol is 70%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 205°C.

2. Preparation of the concentrated pills

The obtained powders are mixed with starch, and the amount of starch is 25% of the weight of the extractum powder. Honey aqueous solution is added as binding agent to produce the blank pills.

3. Coating the concentrated pills

The resultant pills are coated with stomach-soluble Opadry® produced by Shanghai COLORCON Coating Technology Co. Ltd. as the coating materials to give the stomach-soluble coating concentrated pills of the invention.
The coating conditions are as follows: water is selected as solvent, the concentration of coating solution is 20% (w/w), the increase in weight of the pill after coating is 6%, the inlet air temperature is at 90°C, the temperature of tablet bed is at 45°C, the spraying pressure is at 2.50bar, the rotation speed of the coating vessel is at 20rpm and the inlet flow rate is at 4g/min.

Example 12 Preparation of coating pills of the Chinese medicinal compositions of the invention

1. Extraction of the Chinese medicines

The following medicinal materials are taken: 405.6 g of *Radix Angelicae Sinensis,* 405.6 g of *Rhizoma Chuanxiong,* 324.3 g of *Radix Paeoniae alba,* 324.3 g of *Radix Rehmanniae Preparata,* 810.8 g of *Ramulus Uncariae cum*

*Uncis,* 810.8 g of *Caulis Spatholobi,* 810.8 g of *Spica Prunellae,* 810.8 g of *Semen Cassiae,* 810.8 g of *Concha Margaritifera Usta,* 405.6 g of *Rhizoma Corydalis.*

In the above-mentioned ten medicinal materials, *Radix Angelicae Sinensis, Rhizoma Chuanxiong, Radix Paeoniae alba* and *Rhizoma Corydalis* are mixed with 80% ethanol and refluxed for 1.5 hours by heating, the reflux solution is stored for use. The residues are extracted with other five Chinese medicines such as *Radix Rehmanniae Preparata* etc. except *Ramulus Uncariae cum Uncis* for three times, 1.5 hours for each. At the third decoction, the *Ramulus Uncariae cum Uncis* is added. The decoctions are combined, concentrated under reduced pressure till the relative density is 1.10 at 55°C. Ethanol is added to the obtained residue to make the final concentration of ethanol is 70%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 205°C.

2. Preparation of the concentrated pills

The obtained powders are mixed with low-substituted hydroxypropyl cellulose, and the amount of low-substituted hydroxypropyl cellulose is 25% of the weight of the extractum powder. 30% (v/v) ethanol aqueous solution is added as binding agent to produce the blank pills.

3. Coating the concentrated pills

The resultant pills are coated with stomach-soluble Opadry® produced by Shanghai COLORCON Coating Technology Co. Ltd. as the coating materials to give the stomach-soluble coating concentrated pills of the invention.

The coating conditions are as follows: water is selected as solvent, the concentration of coating solution is 20% (w/w), the increase in weight of the pill after coating is 6%, the inlet air temperature is at 90°C, the temperature of tablet bed is at 45°C, the spraying pressure is at 2.50bar, the rotation speed of the coating vessel is at 20rpm and the inlet flow rate is at 4g/min.

Example 13 Preparation of coating pills of the Chinese medicinal compositions of the invention

1. Extraction of the Chinese medicines

The following medicinal materials are taken: 405.6 g of *Radix Angelicae Sinensis,* 405.6 g of *Rhizoma Chuanxiong,* 324.3 g of *Radix Paeoniae alba,* 324.3 g of *Radix Rehmanniae Preparata,* 810.8 g of *Ramulus Uncariae cum Uncis,* 810.8 g of *Caulis Spatholobi,* 810.8 g of *Spica Prunellae,* 810.8 g of *Semen Cassiae,* 810.8 g of *Concha Margaritifera Usta,* 405.6 g of *Rhizoma Corydalis.*

In the above-mentioned ten medicinal materials, *Radix Angelicae Sinensis, Rhizoma Chuanxiong, Radix Paeoniae alba* and *Rhizoma Corydalis* are mixed with 80% ethanol and refluxed for 1.5 hours by heating, the reflux solution is stored for use. The residues are extracted with other five Chinese medicines such as *Radix Rehmanniae Preparata* etc. except *Ramulus* Uncariae cum *Uncis* for three times, 1.5 hours for each. At the third decoction, the *Ramulus Uncariae cum Uncis* is added. The decoctions are combined, concentrated under reduced pressure till the relative density is 1.10 at 55°C. Ethanol is added to the obtained residue to make the final concentration of ethanol is 70%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 205°C.

2. Preparation of the concentrated pills

The obtained powders are mixed with low-substituted hydroxypropyl cellulose, and the amount of low-substituted hydroxypropyl cellulose is 25% of the weight of the extractum powder. 20% (v/v) ethanol aqueous solution is added as binding agent to produce the blank pills.

3. Coating the concentrated pills

The resultant pills are coated with stomach-soluble Opadry® produced by Shanghai COLORCON Coating Technology Co. Ltd. as the coating materials to give the stomach-soluble coating concentrated pills of the invention.

The coating conditions are as follows: water is selected as solvent, the concentration of coating solution is 20% (w/w), the increase in weight of the pill after coating is 6%, the inlet air temperature is at 40°C, the temperature of tablet bed is at 70°C, the spraying pressure is at 2.50bar, the rotation speed of the coating vessel is at 20rpm and the inlet flow rate is at 4g/min.

Example 14 Preparation of the pills of the Chinese medicinal compositions of the invention

1. Extraction of the Chinese medicines

The following medicinal materials are taken: 405.6 g of *Radix Angelicae Sinensis,* 405.6 g of *Rhizoma Chuanxiong,* 324.3 g of *Radix Paeoniae alba,* 324.3 g of *Radix Rehmanniae Preparata,* 810.8 g of *Ramulus Uncariae cum Uncis,* 810.8 g of *Caulis Spatholobi,* 810.8 g of *Spica Prunellae,* 810.8 g of *Semen Cassiae,* 810.8 g of *Concha Margaritifera Usta,* 405.6 g of *Rhizoma Corydalis.*

In the above-mentioned ten medicinal materials, *Radix Angelicae Sinensis, Rhizoma Chuanxiong,* Radix *Paeoniae alba* and *Rhizoma Corydalis* are mixed with 70% ethanol and refluxed for 1 hour by heating, the reflux solution is stored for use. The residues are extracted with other five Chinese medicines such as *Radix Rehmanniae Preparata* etc. except *Ramulus Uncariae cum Uncis* for three times, one hour for each. At the third decoction, the *Ramulus Uncariae cum Uncis* is added. The decoctions are combined, concentrated under reduced pressure till the relative density is 1.10 at 55°C. Ethanol is added to the obtained residue to make the final concentration of ethanol is 65%, allow to stand for 24 hours, filtered the precipitate, and the filtrate and the above-obtained reflux solution are combined, distilled under reduced pressure for recovering ethanol till the relative density is 1.15 at 55°C, and dried to obtain the dried extractum, and further obtain the extractum powder by spray-drying with the inlet air temperature at 185°C.

2. Preparation of the concentrated pills

The obtained powders are mixed with microcrystaline cellulose, and the amount of microcrystaline cellulose is 35% of the weight of the extractum powder. 25% (v/v) ethanol aqueous solution is added as binding agent to produce the blank pills.

**Claims**

1. A Chinese medicinal composition consisting of the following medicinal materials in proportions of weight: 5-15 wt% of *Radix Angelicae Sinensis* (Dang gui), 5-15 wt% of *Rhizoma Chuanxiong* (Chuan xiong), 2-10 wt% of *Radix Paeoniae alba* (Bai shao), 2-10 wt% of *Radix Rehmanni ae Prepara ta* (Shu dihuang), 7-17 wt% of *Ramulus Uncariae cum Uncis* (Gou teng), 7-17 wt% of *Caulis Spatholobi* (Ji Xueteng), 7-17 wt% of *Spica Prunellae* (Xia Kucao), 7-17 wt% of *Semen Cassiae* (Jue mingzi), 7-17 wt% of *Concha Margaritifera Usta* (Zhen zhumu) and 3-10 wt% of *Rhizoma Corydalis* (Yuan hu).

2. The composition according to claim 1, wherein the composition consists of 5-8 wt% of *Radix Angelicae Sinensis,* 5-8 wt% of *Rhizoma Chuanxiong,* 3-7 wt% of *Radix Paeoniae alba,* 3-7 wt% of *Radix Rehmanniae Preparata,* 10-15 wt% of *Ramulus Uncariae cum Uncis,* 10-15 wt% of *Caulis Spatholobi,* 10-15 wt% of *Spica Prunellae,* 10-15 wt% of *Semen Cassiae,* 10-15 wt% of *Concha Margaritifera Usta* and 3-10 wt% of *Rhizoma Corydalis.*

3. The composition according to claim 1, wherein the composition consists of 6.85 wt% of *Radix Angelicae Sinensis,* 6.85 wt% of *Rhizoma Chuanxiong,* 5.5 wt% of *Radix Paeoniae alba,* 5.5 wt% of *Radix Rehmanniae Preparata,* 13.69 wt% of *Ramulus Uncariae cum Uncis,* 13.69 wt% of *Caulis Spatholobi,* 13.69 wt% of *Spica Prunellae,* 13.69 wt% of *Semen Cassiae,* 13.69 wt% of *Concha Margaritifera Usta* and 6.85 wt% of *Rhizoma Corydalis.*

4. The composition according to any of the claims 1 to 3, wherein the composition is prepared to formulations suitable to be administered orally.

5. The composition according to claim 4, wherein the formulations suitable to be administered orally are solid formulations suitable to be administered orally.

6. The composition according to claim 5, wherein the solid formulations suitable to be administered orally are pills.

7. The composition according to claim 6, wherein the pills contain adjuvants and binding agents, the adjuvants are selected from the group consisting of: dextrin, starch, low-substituted hydroxypropyl cellulose and microcrystaline cellulose, the amount of the adjuvants accounts for more than 25% of the weight of dried medicinal materials powder, and the binding agents are selected from the group consisting of:

water, 20-30% (v/v) ethanol aqueous solution, and honey aqueous solution.

8. The composition according to claim 7, wherein the adjuvants in the pills is microcrystaline cellulose, the amount of the adjuvants accounts for 28-35% of the weight of dried medicinal materials powder, and the binding agent is water.

9. A process for preparation of the composition of any one of claims 1 to 8, comprising the following steps:

a. Providing medicinal materials according to the proportions of any one of claims 1 to 3;
b. *Radix Angelicae Sinensis, Rhizoma Chuanxiong, Radix Paeoniae alba* or *Rhizoma Corydalis* is extracted by refluxing with ethanol respectively, the extracts are combined and stored for use; or adding ethanol into a mixture of *Radix Angelicae Sinensis, Rhizoma Chuanxiong, Radix Paeoniae alba* and *Rhizoma Corydalis,* extracting by refluxing with heating, and the reflux solution is stored for use;
c. The residues of step (b) and other five medicinal materials except *Ramulus Uncariae cum Unci* are decocted with water for several times, adding *Ramulus Uncariae cum Unci* in the last decoction; the decoctums are combined and concentrated;
d. The concentrated decoctum is precipitated by addition of ethanol, the obtained supernatant and the reflux solution above-obtained are combined, concentrated by recovering ethanol to give the composition in the extractum form;
e. Mixing the extractum of step (d) with appropriate adjuvants to obtain desired pharmaceutical formulations.

10. The process for preparation of the composition according to claim 9, wherein in step (b), ethanol is added into a mixture of *Radix Angelicae Sinensis, Rhizoma Chuanxiong, Radix Paeoniae alba* and *Rhizoma Corydalis* to extract under reflux by heating; in step (c), the residues of step (b) and other five medicinal materials except *Ramulus Uncariae cum Uncis* are decocted with water for three times; at the third decoction, Ramulus *Uncariae cum Uncis* is added to decoct together, the time for each decoction is 0.5-1.5 hours; the decoctums are combined and concentrated to obtain concentrated extractum with the relative density of 1.09-1.13 at 55°C.

11. The process for preparation of the composition according to claim 10, wherein the concentration of ethanol in the step (b) is 60%-80% (v/v) ; and the time for each reflux is 0.5-1.5 hours; the final content of ethanol in step (d) is 60%-70% (v/v) ; the relative density of the concentrated extractum is about 1.15 at 55°C.

12. The process for preparation of the composition according to any one of claims 9 to 11, wherein the extractum obtained may be dried by spray-dried process to give the extractum powder.

13. The process for preparation of the composition according to any one of claims 9 to 12, wherein the pharmaceutical formulations are film-coating pills, and film-coating materials of the film-coating pills are stomach-soluble film-coating materials, the coating conditions are as follows:

water is selected as solvent, the concentration of coating solution is 14-20% (w/w), the increase in the pill weight after coating is 2%-6%, inlet air temperature is at 40-90°C, the temperature of tablet bed is at 40-70°C, the spraying pressure is at 1.5-2.5bar, the rotation speed of the coating vessel is at 15-20rmp and the inlet flow rate is at 2-4g/min.

14. Use of the composition according to any one of claims 1 to 8 in preparation of drugs for treating headache.

15. The composition according to any of claims 1 to 8 for use in treating headache.

**Patentansprüche**

1. Chinesische medizinische Zusammensetzung, bestehend aus den folgenden medizinischen Materialien in Gewichtsanteilen: 5 - 15 Gew.-% *Radix Angelicae Sinensis* (Dang gui), 5 - 15 Gew.-% *Rhizoma Chuanxiong* (Chuan xiong), 2 - 10 Gew.-% *Radix Paeoniae alba* (Bai shao), 2 - 10 Gew.-% *Radix Rehmanniae Preparata* (Shu dihuang), 7 - 17 Gew.-% *Ramulus Uncariae cum Uncis* (Gou teng), 7-17 Gew.- % Caulis Spatholobi (Ji Xueteng), 7 - 17 Gew.-% *Spica Prunellae* (Xia Kucao), 7 - 17 Gew.-% *Semen Cassiae* (Jue mingzi), 7 - 17 Gew.-% *Concha Margaritifera Usta* (Zhen zhumu) und 3 - 10 Gew.-% *Rhizoma Corydalis* (Xuan hu).

2. Zusammensetzung nach Anspruch 1, wobei die Zusammensetzung besteht aus 5 - 8 Gew.-% *Radix Angelicae*

*Sinensis,* 5 - 8 Ges.-% *Rhizoma Chuanxiong,* 3 - 7 Gew.-% *Radix Paeoniae alba,* 3 - 7 Gew.-% *Radix Rehmanniae Preparata,* 10 - 15 Gew.-% *Ramulus Uncariae cum Uncis,* 10 - 15 Gew.-% *Caulis Spatholobi,* 10 - 15 Gew.-% *Spica Prunellae,* 10 - 15 Gew.-% *Semen Cassiae,* 10 - 15 Gew.-% *Concha Margaritifera Usta* und 3 - 10 Gew.-% von *Rhizoma Corydalis.*

**3.** Zusammensetzung nach Anspruch 1, wobei die Zusammensetzung besteht aus 6,85 Gew.-% *Radix Angelicae Sinensis,* 6,85 Gew.-% *Rhizoma Chuanxiong,* 5,5 Gew.-% *Radix Paeoniae alba,* 5,5. Gew.-% *Radix Rehmanniae Preparata,* 13,69 Gew.-% *Ramulus Uncariae cum Uncis,* 13,69 Gew.-% *Caulis Spatholobi,* 13,69 Gew.-% *Spica Prunellae,* 13,69 Gew.-% *Semen Cassiae,* 13,69 Gew.-% *Concha Margaritifera Usta* und 6,85 Gew.-% von *Rhizoma Corydalis.*

**4.** Zusammensetzung nach einem der Ansprüche 1 bis 3, wobei die Zusammensetzung hergestellt ist für Formulierungen, die zur oralen Verabreichung geeignet sind.

**5.** Zusammensetzung nach Anspruch 4, wobei die Formulierungen, die geeignet sind, oral verabreicht zu werden, feste Formulierungen sind, die geeignet sind, oral verabreicht zu werden.

**6.** Zusammensetzung nach Anspruch 5, wobei die festen Formulierungen, die geeignet sind, oral verabreicht zu werden, Pillen sind.

**7.** Zusammensetzung nach Anspruch 6, wobei die Pillen Adjuvantien und Bindemittel enthalten, wobei die Adjuvantien gewählt sind aus der Gruppe, die besteht aus: Dextrin, Stärke, niedersubstituierter Hydroxypropylzellulose und mikrokristalliner Zellulose, wobei die Menge der Adjuvantien mehr als 25 Gew.-% getrockneter medizinischer Pulvermaterialien ausmacht und die Bindemittel gewählt sind aus der Gruppe, die besteht aus: Wasser, 20 - 30%iger (v/v) wäßriger Ethanollösung und wäßriger Honiglösung.

**8.** Zusammensetzung nach Anspruch 7, wobei die Adjuvantien in den Pillen mikrokristalline Zellulose sind, wobei die Menge der Adjuvantien 28 - 35 Gew.-% getrockneter medizinischer Pulvermaterialen ausmacht und das Bindemittel Wasser ist.

**9.** Verfahren zur Herstellung der Zusammensetzung nach einem der Ansprüche 1 bis 8, umfassend die folgenden Schritte:

a. Bereitstellen medizinischer Materialien in den Anteilen gemäß einem der Ansprüche 1 bis 3;

b. *Radix Angelicae Sinensis, Rhizoma Chuanxiong, Radix Paeoniae alba* oder *Rhizoma Corydalis* wird extrahiert durch Refluxieren mit jeweils Ethanol, die Extrakte werden vereint und zur Verwendung gelagert; oder Zugeben von Ethanol in ein Gemisch von *Radix Angelicae Sinensis, Rhizoma Chuanxiong, Radix Paeoniae alba* und *Rhizoma Corydalis,* Extrahieren durch Refluxieren unter Erhitzen und die Rückflußlösung wird zur Verwendung gelagert;

c. die Rückstände von Schritt (b) und anderen fünf medizinischen Materialien außer *Ramulus Uncariae cum Unci werden* mit Wasser mehrmals abgesiedet, wobei beim letzten Absieden *Ramulus Uncariae cum Unci* zugegeben wird; die Absiedungen werden vereint und konzentriert;

d. die konzentrierte Absiedung wird gefällt durch Zugabe von Ethanol, der erhaltene Überstand und die oben erhaltene Rückflußlösung werden vereint, konzentriert unter Rückgewinnung von Ethanol, um die Zusammensetzung in der Extraktform zu ergeben;

e. Vermischen der Extrakte von Schritt (d) mit geeigneten Adjuvantien, um gewünschte pharmazeutische Formulierungen zu erhalten.

**10.** Verfahren zur Herstellung der Zusammensetzung nach Anspruch 9, wobei in Schritt (b) Ethanol in ein Gemisch zugegeben wird von *Radix Angelicae Sinensis, Rhizoma Chuanxiong, Radix Paeoniae alba* und *Rhizoma Corydalis,* um unter Rückfluß durch Erhitzen zu extrahieren; in Schritt (c) die Rückstände von Schritt (b) und anderen fünf medizinischen Materialien außer *Ramulus Uncariae cum Uncis* mit Wasser dreimal abgesiedet werden; bei der dritten Absiedung *Ramulus Uncariae cum Uncis* zugegeben wird, um zusammen abzusieden, wobei die Zeit für jede Absiedung 0,5 bis 1,5 Stunden ist; die Absiedungen werden vereint und konzentriert, um konzentriertes Extrakt mit der relativen Dichte von 1,09 - 1,13 bei 55 °C zu erhalten.

**11.** Verfahren zur Herstellung der Zusammensetzung nach Anspruch 10, wobei die Ethanolkonzentration in Schritt (b) 60 - 80 % (v/v) ist und die Zeit für jedes Refluxieren 0,5 bis 1,5 Stunden ist; der Endethanolgehalt in Schritt (d) 60 - 70 % (v/v) ist; die relative Dichte des konzentrierten Extraktes etwa 1,15 bei 55 °C ist.

**12.** Verfahren zur Herstellung der Zusammensetzung nach einem der Ansprüche 9 bis 11, wobei die erhaltenen Extrakte getrocknet werden können durch ein Sprühtrocknungsverfahren, um das Extraktpulver zu ergeben.

**13.** Verfahren zur Herstellung der Zusammensetzung nach einem der Ansprüche 9 bis 12, wobei die pharmazeutischen Formulierungen filmbeschichtete Pillen sind und Filmbeschichtungsmaterialien der filmbeschichteten Pillen magenlösliche Filmbeschichtungsmaterialien sind, wobei die Beschichtungsbedingungen wie folgt sind: Wasser wird als Lösungsmittel gewählt, die Konzentration der Beschichtungslösung beträgt 14 - 20 % (w/w), die Vergrößerung des Pillengewichts nach Beschichtung beträgt 2 - 6 %, die Einlasslufttemperatur beträgt 40 - 90 °C, die Temperatur des Tablettenbetts beträgt 40 - 70 °C, der Sprühdruck beträgt 1,5 - 2,5 bar, die Rotationsgeschwindigkeit des Beschichtungsgefäßes beträgt 15 - 20 U/min und die Einlaßflußgeschwindigkeit liegt bei 2 - 4 g/min.

**14.** Verwendung der Zusammensetzung nach einem der Ansprüche 1 bis 8 bei der Herstellung von Medikamenten zur Behandlung von Kopfschmerzen.

**15.** Zusammensetzung nach einem der Ansprüche 1 bis 8 zur Verwendung bei der Behandlung von Kopfschmerzen.

## Revendications

**1.** Composition médicinale chinoise consistant en les substances médicinales suivantes en les proportions en poids : 5-15 % en poids de *Radix Angelicae Sinensis* (Dang gui), 5-15 % en poids de *Rhizoma Chuanxiong* (Chuan xiong), 2-10 % en poids de *Radix Paeoniae alba* (Bai shao), 2-10 % en poids de *Radix Rehmanniae Preparata* (Shu dihuang), 7-17 % en poids de *Ramulus Uncariae cum Uncis* (Gou teng), 7-17 % en poids de *Caulis Spatholobi* (Ji Xueteng), 7-17 % en poids de *Spica Prunellae* (Xia Kucao), 7-17 % en poids de *Semen Cassiae* (Jue mingzi), 7-17 % en poids de *Concha Margaritifera Usta* (Zhen zhumu) et 3-10 % en poids de *Rhizoma Corydalis (Yuan* hu).

**2.** Composition selon la revendication 1, où la composition consiste en 5-8 % en poids de *Radix Angelicae Sinensis,* 5-8 % en poids de *Rhizoma Chuanxiong,* 3-7 % en poids de *Radix Paeoniae alba,* 3-7 % en poids de *Radix Rehmanniae Preparata,* 10-15 % en poids de *Ramulus Uncariae cum Uncis,* 10-15 % en poids de *Caulis Spatholobi,* 10-15 % en poids de *Spica Prunellae,* 10-15 % en poids de *Semen Cassiae,* 10-15 % en poids de *Concha Margaritifera Usta* et 3-10 % en poids de *Rhizoma Corydalis*

**3.** Composition selon la revendication 1, où la composition consiste en 6,85 % en poids de *Radix Angelicae Sinensis,* 6,85 % en poids de *Rhizoma Chuanxiong,* 5,5 % en poids de *Radix Paeoniae alba,* 5,5 % en poids de *Radix Rehmanniae Preparata,* 13,69 % en poids de *Ramulus Uncariae cum Uncis,* 13,69 % en poids de *Caulis Spatholobi,* 13,69 % en poids de *Spica Prunellae,* 13,69 % en poids de *Semen Cassiae,* 13,69 % en poids de *Concha Margaritifera Usta* et 6,85 % en poids de *Rhizoma Corydalis.*

**4.** Composition selon l'une quelconque des revendications 1 à 3, où la composition est préparée en formulations appropriées pour être administrées oralement.

**5.** Composition selon la revendication 4, où les formulations appropriées pour être administrées oralement sont des formulations solides appropriées pour être administrées oralement.

**6.** Composition selon la revendication 5, où les formulations solides appropriées pour être administrées oralement sont des pilules.

**7.** Composition selon la revendication 6, où les pilules contiennent des adjuvants et des agents liants, les adjuvants sont choisis dans le groupe consistant en : la dextrine, l'amidon, l'hydroxypropyl-cellulose faiblement substituée et la cellulose microcristalline, la quantité des adjuvants représente plus de 25 % du poids de la poudre de substances médicinales séchées, et les agents liants sont choisis dans le groupe consistant en : l'eau, une solution aqueuse d'éthanol à 20-30 % (v/v) et une solution aqueuse de miel.

**8.** Composition selon la revendication 7, où les adjuvants dans les pilules sont la cellulose microcristalline, la quantité des adjuvants représente 28-35 % du poids de la poudre de substances médicinales séchées, et l'agent liant est l'eau.

**9.** Procédé pour la préparation de la composition selon l'une quelconque des revendications 1 à 8, comprenant les étapes suivantes :

   a. fourniture de substances médicinales selon les proportions de l'une quelconque des revendications 1 à 3 ;
   b. *Radix Angelicae Sinensis, Rhizoma Chuanxiong, Radix Paeoniae alba ou Rhizoma Corydalis* est extrait par reflux avec l'éthanol respectivement, les extraits sont combinés et stockés pour l'utilisation ; ou addition d'éthanol dans un mélange de *Radix Angelicae Sinensis, Rhizoma Chuanxiong, Radix Paeoniae alba* et *Rhizoma Corydalis,* extraction par reflux avec chauffage, et la solution de reflux est stockée pour l'utilisation ;
   c. les résidus de l'étape (b) et cinq autres substances médicinales sauf *Ramulus Uncariae cum Unci* sont soumis plusieurs fois à une décoction avec de l'eau, addition de *Ramulus Uncariae cum Unci* dans la dernière décoction ; les produits de décoction sont combinés et concentrés ;
   d. le produit de décoction concentré est précipité par addition d'éthanol, le surnageant obtenu et la solution de reflux obtenue ci-dessus sont combinés, concentrés par récupération de l'éthanol pour donner la composition sous forme d'extrait ;
   e. mélange de l'extrait de l'étape (d) avec des adjuvants appropriés pour obtenir des formulations pharmaceutiques souhaitées.

**10.** Procédé pour la préparation de la composition selon la revendication 9 où, dans l'étape (b), de l'éthanol est ajouté dans un mélange de *Radix Angelicae Sinensis, Rhizoma Chuanxiong, Radix Paeoniae alba* et *Rhizoma Corydalis* pour extraire à reflux par chauffage ; dans l'étape (c), les résidus de l'étape (b) et cinq autres substances médicinales sauf *Ramulus Uncariae cum Uncis* sont soumis à une décoction avec de l'eau trois fois ; à la troisième décoction, *Ramulus Uncariae cum Uncis* est ajouté en vue d'une décoction commune, la durée pour chaque décoction est 0,5-1,5 heure ; les produits de décoction sont combinés et concentrés pour obtenir un extrait concentré ayant une densité relative de 1,09-1,13 à 55°C.

**11.** Procédé pour la préparation de la composition selon la revendication 10, où la concentration de l'éthanol dans l'étape (b) est 60 %-80 % (v/v) ; et la durée pour chaque reflux est 0,5-1,5 heure ; la teneur finale de l'éthanol dans l'étape (d) est 60 %-70 % (v/v) ; la densité relative de l'extrait concentré est d'environ 1,15 à 55°C.

**12.** Procédé pour la préparation de la composition selon l'une quelconque des revendications 9 à 11, où l'extrait obtenu peut être séché par un procédé de séchage par pulvérisation pour donner la poudre d'extrait.

**13.** Procédé pour la préparation de la composition selon l'une quelconque des revendications 9 à 12, où les formulations pharmaceutiques sont des pilules enrobées d'un film, et les substances d'enrobage avec un film des pilules enrobées d'un film sont des substances d'enrobage avec un film solubles dans l'estomac, les conditions de l'enrobage sont les suivantes : l'eau est choisie comme solvant, la concentration de la solution d'enrobage est 14-20 % (p/p), l'augmentation du poids des pilules après l'enrobage est 2 %-6 %, la température de l'air d'entrée est à 40-90°C, la température du lit de comprimés est à 40-70°C, la pression de pulvérisation est à 1,5-2,5 bar, la vitesse de rotation du récipient d'enrobage est à 15-20 tr/min et le débit d'entrée est à 2-4 g/min.

**14.** Utilisation de la composition selon l'une quelconque des revendications 1 à 8 dans la préparation de médicaments pour traiter les céphalées.

**15.** Composition selon l'une quelconque des revendications 1 à 8 destinée à être utilisée dans le traitement des céphalées.

Cumulative release percentage

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN ZL93100050
- CN 20041201

**Non-patent literature cited in the description**

- **Ding Tao.** The adverse reaction of Chinese medicine and its prevention [M]. China Press of Chinese Medicine, 1992, 85